# EUROPEAN PATENT SPECIFICATION

(11) **EP 0 579 840 B1**
(45) Date of publication and mention of the grant of the patent: **09.08.1995**
(21) Application number: 93902528.4
(22) Date of filing: 26.01.1993
(51) Int. Cl.: C07C 401/00, A61K 31/59, C07F 9/53, C07F 7/18

(54) **FLUORINE-CONTAINING VITAMIN D3 ANALOGUES**
Fluor-enthaltende Vitamin-D3-Analogen
ANALOGUES DE VITAMINE D3 CONTENANT DU FLUOR

(30) Priority: 10.02.1992 US 832888
(43) Date of publication of application: 26.01.1994
(73) Proprietor: DAIKIN INDUSTRIES, LTD., Kita-ku, Osaka 530 (JP)
(72) Inventor: IKEKAWA, Nobuo 2-21-5, Kichijojihigashi-cho, Tokyo 180 (JP); TAGUCHI, Takeo 3-14-8-103, Minamiosawa, Tokyo 192-03 (JP); TANAKA, Yoko, Delmar, NY 12054 (US); KOBAYASHI, Yoshiro 6-11-1009, Udagawa-cho, Tokyo 150 (JP); OHIRA, Yutaka 3, Miyukigaoka, Ibaraki 305 (JP)
(74) Representative: Weisert, Annekäte, Dipl.-Ing. Dr.-Ing.
(86) International application number: PCT/JP93/00088
(87) International publication number: WO 93/16040

(56) References cited:
- EP-A- 0 326 875
- CHEMICAL AND PHARMACEUTICAL BULLETIN. vol. 40, no. 6, June 1992, TOKYO JP pages 1647 - 1649 Y. OHIRA ET AL 'Preparation of (22S)- and (22R)-24-Homo-26,26,26,27,27,27-Hexafluoro -1,22,25-Trihydroxyvitamin D3'

## Description

This invention relates to novel fluorine-containing vitamin D₃ analogues which have excellent pharmacological activities, such as tumor cell differentiation-inducing activity and are expected to be used as a medicament.

### Background Art

It is known that a bio-metabolite of vitamin D₃, 1α,25-dihydroxyvitamin D₃ is called as "active-type vitamin D₃" and has an activity of promoting absorption of calcium via intestinal tract and thereby is useful as a medicament for the treatment of bone diseases. Recently, it has been found that the active-type vitamin D and analogues thereof have a differentiation-inducing activity for recovering normal cells from cancerated cells (cf. Hirobumi Tanaka et al., "Seikagaku" (Biochemistry), Vol. 55, 1323, 1983) and further that some of these compounds have a remarked activity of inhibiting the progress of cancer (K.W. Colton et al., Lancet, Jan. 28, 188, 1989). It has, however, been known that these active-type vitamin D compounds have high antagonistic activity against calcium metabolism which induces hypercalcaemia and hence can not be used in a high dose. Accordingly, these compounds are not necessarily usable for the treatment of diseases which require continuous administration in a comparatively high dose, for example, for the treatment of leukemia.

### Disclosure of Invention

The present inventors have intensively studied as to novel fluorine-containing vitamin D₃ analogues which have excellent cell differentiation-inducing activity as well as high selectivity in calcium metabolism with less side effects, i.e. inhibition of hepercalcaemia.

An object of the invention is to provide novel fluorine-containing vitamin D₃ analogues having pharmacological activities, especially anti-tumor activity owing to the cell differentiation-inducing activity. A further object of the invention is to provide a novel intermediate suitable for the preparation of the active fluorine-containing vitamin D₃ analogues. These and other objects and advantages of the invention will be apparent to the skilled persons in this field from the following description.

The fluorine-containing vitamin D₃ analogues of this invention have the following formula [I]: 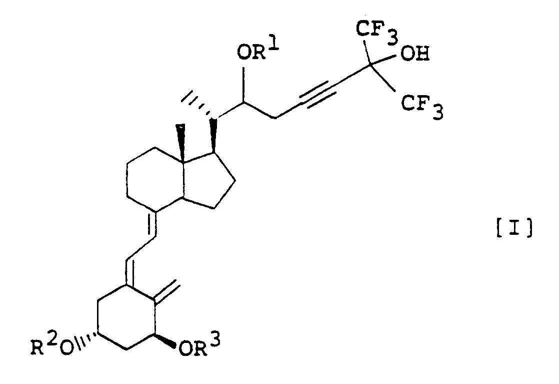
wherein R¹, R² and R³ are independently hydrogen atom or a hydroxy-protecting group.

In the present specification and claims, the hydroxy-protecting group includes a group being capable of forming acetal type protecting group (e.g. methoxymethyl, ethoxyethyl, methoxyethoxymethyl, tetrahydropyranyl, etc.), a silyl ether type protecting group (e.g. trimethylsilyl, t-butyldimethylsilyl, t-butyldiphenylsilyl, etc.), an acyl group (e.g. acetyl), and the like.

Suitable examples of the compounds [I] are as follows.
1) Compound A: 26,26,26,27,27,27-Hexafluoro-24-homo-24-yne-1α,22S,25-trihydroxyvitamin D₃ 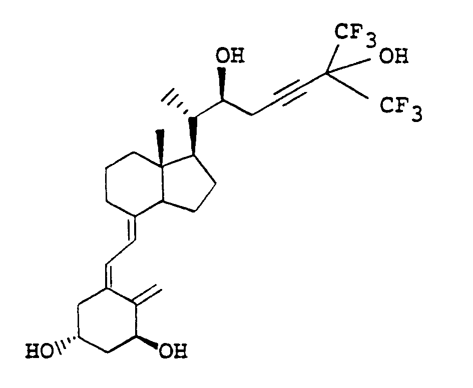
2) Compound B: 26,26,26,27,27,27-Hexafluoro-24-homo-24-yne-1α,22R,25-trihydroxyvitamin D₃ 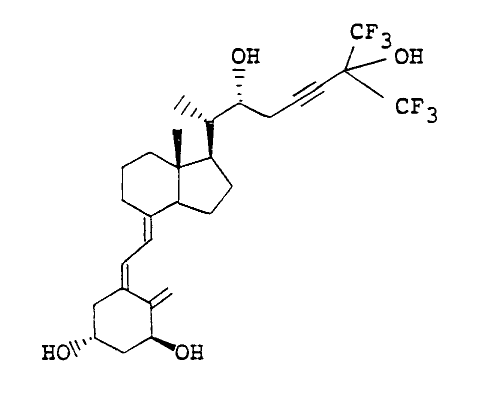
3) 1α,3-Bis(t-butyldimethylsilyl) ether 22-acetate of Compound A
4) 1α,3-Bis(t-butyldimethylsilyl) ether 22-acetate of Compound B
The compounds [I] of this invention can be prepared by various processes. One of the best processes is illustrated below.

A [ring C,D] fragment of the formula [II]: 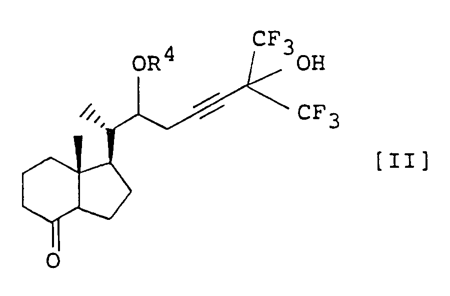
wherein R⁴ is a hydroxy-protecting group, is subjected to coupling reaction with an anion derived from a protected [ring A] fragment of the formula [III]: 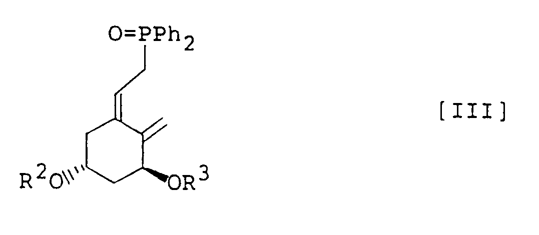
wherein R² and R³ are each a hydroxy-protecting group, and Ph means phenyl, to give a condensed product of the formula [I] of this invention.

The above coupling reaction of the compound [II] and the compound [III] is usually carried out at a low temperature, for example -100°C to -50°C, preferably -78°C to -20°C, in an ether solvent (e.g. diethyl ether, tetrahydrofuran (THF), etc.). The conversion of the [ring A] fragment into the corresponding carbanion is carried out by treating the fragment with an appropriate base such as an alkyl-lithium (e.g. n-butyllithium, etc). The reaction time is for 10 minutes to 24 hours, preferably for 30 minutes to 2 hours. The obtained product [I] can be purified by a conventional method, for example, by silica gel column chromatography. The removal of the hydroxy-protecting group from the compound [I] can optionally be carried out by a conventional method.

Starting compound [II) can be prepared by the process as illustrated by the following reaction scheme: 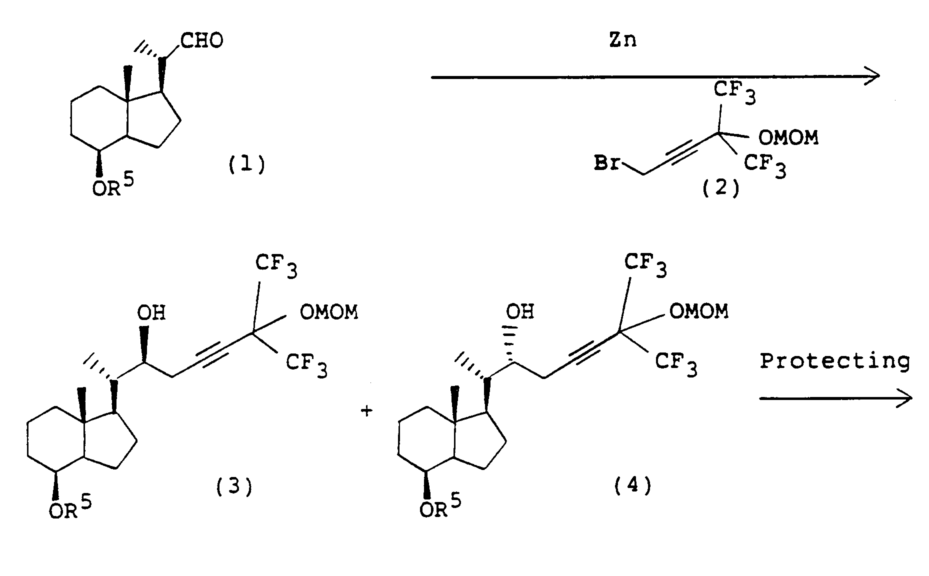 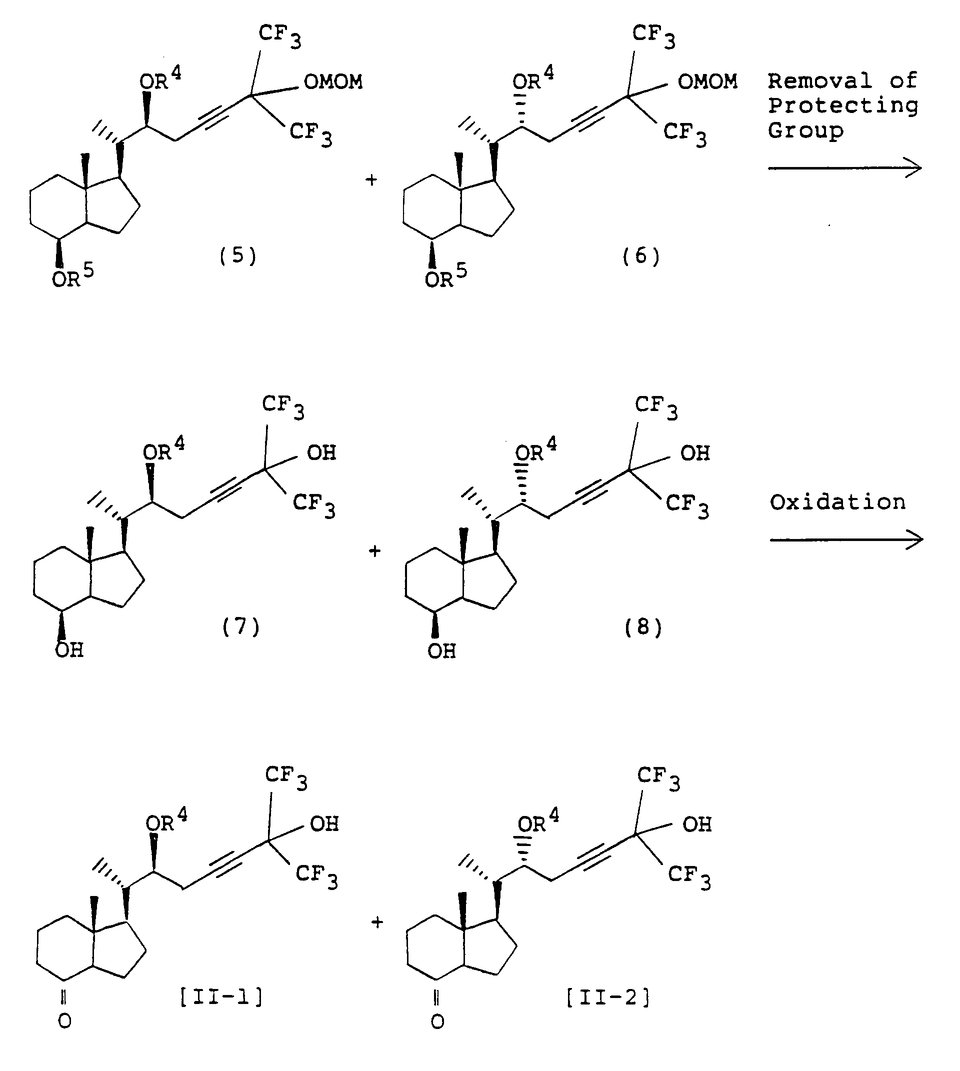
wherein R⁴ and R⁵ are each a hydroxy-protecting group, and MOM means methoxymethyl group.

According to the above process, the starting compound [II] ([II-1] and [11-2]) can be prepared by reacting the aldehyde compound (1) with the bromide compound (2) to give the compounds (3) and (4), protecting the hydroxy group of the resultant compounds (3) and (4) with a hydroxy-protecting group in a usual manner, removing the hydroxy-protecting group R⁵ from the resultant compounds (5) and (6), and finally oxdizing the resultant compounds (7) and (8).

### Best Mode for Carrying Out of the Invention

The compounds of this invention are illustrated by the following Examples and Reference Examples, but should not be constructed to be limited thereto.

### Example 1

### 1-1) Preparation of 1α,3-bis(t-butyldimethylsilyl) ether of compound (A) by Wittig reaction of compound [II-1] wherein R⁴ is acetyl and compound [III] wherein R² and R³ are t-butyldimethylsilyl:

To a solution of the compound [III] wherein R² and R³ are t-butyldimethylsilyl (1.0 g) in anhydrous THF (10 ml) is added n-BuLi (2.5 M, 0.68 ml) at -78°C, and the mixture is stirred for 5 minutes. To the solution is added a solution of the compound [II-1] wherein R⁴ is acetyl (80 mg) in anhydrous THF (5 ml) is added and the mixture is stirred for 10 minutes after warming to room temperature. To the reaction mixture is poured a saturated ammonium chloride solution and the mixture is extracted with ethyl acetate. The ethyl acetate layer is washed with water and dried over anhydrous magnesium sulfate. After distilling off the solvent, the residue is purified by column chromatography to give the desired compound (106.9 mg, 78 %) as a colorless solid.
¹H-NMR (CDCl₃) δ: 0.05 (s, 6H), 0.06 (s, 6H), 0.54 (s, 3H), 0.866 (s, 9H), 0.875 (s, 9H), 0.94 (d, J=7.1Hz, 3H), 4.86 (d, J=2.8Hz, 1H), 5.18 (d, J=2.8Hz, 1H), 6.03 (d, J=12.2Hz, 1H), 6.24 (d, J=12.2Hz, 1H)
IR (KBr): 3431, 2954, 1221, 834 cm⁻¹ 1-2) Preparation of compound (A) by removal of the protecting silyl group:
The silyl compound obtained in Example 1-1 (99 mg) is added to a suspension of ion-exchange resin (50W×4, 3 g) in methanol (30 ml) and stirred for 24 hours at room temperature. After filtering the solution and distilling off the solvent, the residue is purified by column chromatography to give the desired compound (A) (66 mg).
¹H-NMR (CDCl₃) δ: 0.58 (s, 3H), 0.91 (d, J=5.6Hz, 3H), 3.85-4.40 (m, 3H), 4.91 (brs, 1H), 5.29 (brs, 1H), 6.11 (d, J=12Hz, 1H), 6.34 (d, J=12Hz, 1H)
IR (KBr): 3383, 2948, 1221, 858 cm⁻¹

### Example 2

### Preparation of compound (B) from compound [II-2] wherein R⁴ is acetyl:

Following the procedure of Example 1 except substituting compound [II-2] for compound [II-1], the desired compound (B) is obtained as a colorless solid.
¹H-NMR (CDCl₃) δ: 0.56 (s, 3H), 0.95 (d, J=6Hz, 3H), 2.59 (dd, J=11Hz, 3Hz), 2.85 (dd, J=11Hz, 3Hz), 3.94 (m, 1H), 4.23 (m, 1H), 4.43 (m, 1H), 5.00 (brs, 1H), 5.33 (brs, 1H), 6.02 (d, J=11Hz, 1H), 6.37 (d, J=11Hz, 1H)

### Reference Example 1

### Preparation of compound (3) and (4) wherein R⁵ is t-butyldimethylsilyl by reacting compound (1) wherein R⁵ is t-butyldimethylsilyl with compound (2):

To a solution of the aldehyde compound (1) wherein R⁵ is t-butyldimethylsilyl group (1.14 g) and the bromide compound (2) (2.30 g) in DMF (8 ml) is added zinc powder (0.59 g) at 25°C and the mixture is stirred for 30 minutes. After adding a saturated ammonium chloride solution, the mixture is extracted with ether. The ether layer is washed with water and dried over anhydrous magnesium sulfate. After distilling off the solvent, the residue is purified by column chromatography to give the desired compound (3) (1.36 g) and the compound (4) wherein R⁵ is t-butyldimethylsilyl (0.54 g).

As to compound (3): ¹H-NMR (CDCl₃) δ: 0.00 (s, 3H), 0.01 (s, 3H), 0.01 (s, 3H), 0.90 (s, 9H), 0.91 (d, J=5Hz, 3H), 0.91 (s, 3H), 2.32 (dd, J=17Hz, 5Hz, 1H), 2.60 (dd, J=17Hz, 9Hz, 1H), 3.47 (s, 3H), 3.95 (m, 1H), 4.00 (brs, 1H), 5.07 (d, J=25Hz, 1H), 5.09 (d, 25Hz, 1H)
IR (KBr): 3470, 2250, 1230 cm⁻¹
As to compound (4): ¹H-NMR (CDCl₃) δ: 0.00 (s, 3H), 0.01 (s, 3H), 0.89 (s, 9H), 0.93 (d, J=5Hz, 3H), 0.95 (s, 3H), 3.48 (s, 3H), 3.89 (m, 1H), 4.00 (brs, 1H), 5.08 (d, J=25Hz, 1H), 5.10 (d, J=25Hz, 1H)
IR (CHCl₃): 3520, 2260, 1230 cm⁻¹

### Reference Example 2

### Preparation of compound (5) wherein R⁴ is acetyl and R⁵ is t-butyldimethylsilyl by protecting compound (3) obtained in Reference Example 1:

A solution of the alcohol compound (3) obtained in Reference Example 1 (149 mg), acetic anhydride (0.7 ml), pyridine (1.2 ml) and 4-dimethylaminopyridine (35 mg) in dichloromethane (2.5 ml) is stirred for 18 hours at room temperature. After completing the reaction, the mixture is extracted with ether, the ether extract is washed with 2% HCl, 5% sodium bicarbonate solution and brine. After distilling off the solvent, the residue is purified by column chromatography to give the desired compound (5) wherein R⁴ is acetyl and R⁵ is t-butyldimethylsilyl (140 mg).
¹H-NMR (CDCl₃) δ: 0.00 (s, 6H), 0.88 (s, 9H), 0.90 (s, 3H), 0.96 (d, J=6.8Hz, 3H), 2.05 (s, 3H), 3.43 (s, 3H), 3.98 (brs, 1H), 5.03 (s, 2H), 5.08 (m, 1H)
IR (neat): 2956, 2256, 1747, 1472, 1376 cm⁻¹

### Reference Example 3

### Preparation of compound (6) wherein R⁴ is acetyl and R⁵ is t-butyldimethylsilyl by protecting compound (4) obtained in Reference Example 1:

Following the procedure of Reference Example 2 except substituting the compound (4) obtained in Reference Example 1 for the compound (3), the desired compound (6) wherein R⁴ is acetyl and R⁵ is t-butyldimethylsilyl is obtained.
¹H-NMR (CDCl₃) δ: 0.00 (s, 3H), 0.01 (s, 3H), 0.88 (s, 9H), 0.92 (s, 3H), 0.93 (d, J=7Hz, 3H), 2.03 (s, 3H), 2.53 (m, 2H), 3.43 (s, 3H), 4.01 (brs, 1H), 5.02 (d, J=25Hz, 1H), 5.04 (d, J=25Hz, 1H), 5.11 (m, 1H)
Melting Point: 74.3°C to 75.5°C (ethanol)

### Reference Example 4

### Preparation of compound (7) wherein R⁴ is acetyl by removal of the protecting group of compound (5) obtained in Reference Example 2:

A mixture of the acetate compound (5) obtained in reference Example 2 (200 mg), dichloromethane (2.4 ml), acetic acid (2.4 ml) and 5% HCl (0.4 ml) is refluxed for 5 hours. After completing the reaction, the mixture is extracted with ethyl acetate, and the extract is washed with 5% sodium bicarbonate solution and dried over magnesium sulfate. After distilling off the solvent, the residue is purified by column chromatography to give the desired compound (7) wherein R⁴ is acetyl (65 mg, 44%).
¹H-NMR (CDCl₃) δ: 0.94 (s, 3H), 0.98 (d, J=6.8Hz, 3H), 2.09 (s, 3H), 4.09 (brs, 1H), 4.77 (s, 1H), 5.23 (m, 1H)
IR (KBr): 3545, 3219, 2937, 1719, 1250, 1200, 958 cm⁻¹

### Reference Example 5

### Preparation of compound (8) wherein R⁴ is acetyl by removal of the protecting group of compound (6) obtained in Reference Example 3:

Following the procedure of Reference Example 4 except substituting the compound (6) obtained in Reference Example 3 for the compound (5), the desired compound (8) wherein R⁴ is acetyl is obtained.
¹H-NMR (CDCl₃) δ: 0.96 (s, 3H), 0.97 (d, J=7Hz, 3H), 2.07 (s, 3H), 2.46 (m, 2H), 4.09 (brs, 1H), 5.20 (m, 1H)
Melting point: 156°C to 157.5°C (ether/hexane)

### Reference Example 6

### Preparation of compound [II-1] wherein R⁴ is acetyl by oxidation of compound (7) obtained in Reference Example 4:

To a solution of pyridinium chlorochromate (PCC, 50 mg) in dichloromethane (2 ml) is added a solution of alcohol compound (7) obtained in Reference Example 4 (21 mg) in dichloromethane (2 ml), and the mixture is stirred for 4 hours at room temperature. After adding ether, the mixture is filtered. After distilling off the solvent of the filtrate, the residue is purified by column chromatography to give the desired compound [II-1] wherein R⁴ is acetyl (18.9 mg, 91%).
¹H-NMR (CDCl₃) δ: 0.64 (s, 3H), 1.04 (d, J=6.6Hz, 3H), 2.10 (s, 3H), 4.53 (brs, 1H), 5.22 (m, 1H)
IR (neat): 3262, 2964, 2252, 1738, 1713, 1698, 1240, 957 cm⁻¹

### Reference Example 7

### Preparation of compound [II-2] wherein R⁴ is acetyl by oxidation of compound (8) obtained in Reference Example 5:

Following the procedure of Reference Example 6 except substituting the compound (8) obtained in Reference Example 5 for the compound (7), the desired compound [II-2] is obtained.
¹H-NMR (CDCl₃) δ: 0.65 (s, 3H), 1.05 (d, J=7Hz, 3H), 3.26 (brs, 1H), 5.35 (dt, J=15Hz, 1H), 5.45 (dd, J=15Hz, 5Hz, 1H)

### Experiment 1

### Effects of the compound on differentiation-inducing activity:

### Test method:

Subculture cells (HT-29) derived from human colonic cancer were inoculated onto a 24-well plate for tissue culture and was cultured in RPMI-1640 medium (added with 10 % fetal calf serum). After culturing for about 24 hours, the supernatant was removed. To the residue was added a medium containing 2 x 10⁻³M sodium butyrate and the test compound as mentioned below (exchange of the medium), and the mixture was subjected to station culture in a culture vessel containing carbon dioxide (5 % CO₂ - 95 % air) at 37°C. On every other day, the culture medium was exchanged with the same medium as mentioned above, and on 7th day, the number of the mucin-producing cells and shape of the cells were observed by the method of Augeron et al. [cf. Cancer Res, Vol. 44, 3961, 1984].

It is known that the mucin-production is observed in normal cells of large intestine (the colon) but not in cancerated HT-29 cells. Accordingly, as a marker for measuring the fact that the cancer cells HT-29 was differentiated and could express characteristic of normal cells, the number of mucin-producing cells was measured.

### Test compounds:

1. 1α,25-dihydroxyvitamin D₃
2. Compound A: 26,26,26,27,27,27-Hexafluoro-24-homo-24-yne-1α,22S,25-trihydroxyvitamin D₃
3. Compound B: 26,26,26,27,27,27-Hexafluoro-24-homo-24-yne-1α,22R,25-trihydroxyvitamin D₃

### Results:

The data obtained above were shown in percentage based on whole cells (200 cells) measured. The results are shown in the following Table 1.

**Table 1**

| Test compound | Concentration (M) | Number of mucin-producing cells (%) |
|---|---|---|
| Non | 0 | 3 ± 3 |
| 1α,25-dihydroxyvitamin D₃ | 10⁻⁷ | 100 |
| 1α,25-dihydroxyvitamin D₃ | 10⁻⁸ | 39 |
| Compound A | 10⁻⁷ | 97 ± 3 |
| Compound A | 10⁻⁸ | 79 ± 7 |
| Compound A | 10⁻⁹ | 26 ± 9 |
| Compound B | 10⁻⁷ | 90 ± 10 |
| Compound B | 10⁻⁸ | 91 ± 9 |
| Compound B | 10⁻⁹ | 49 ± 8 |

As is clear from the above results, when the HT-29 cells were treated by 2 x 10⁻³M sodium butyrate and the compounds of this invention, the cells were differentiated into mucin-producing cells.

### Experiment 2

### Effects of the compounds on serum calcium concentration:

### Test Method:

In accordance with the method of Mori et al [Vitamin-gaku Jikken-ho (Experiments in Vitamin Science), [I], Fat Soluble Vitamins, Ed. by Japan Vitamin Association, issued by Tokyo Kagaku Dojin, pages 120-135], Vitamin D-defficient rats were prepared and the calcium concentration in serum was measured.

That is, Wistar rats (5 rats per each group) was fed with vitamin D-free, low calcium (0.02 %) feed for about 3 weeks.

After confirming the low calcium concentration in serum (less than 6 mg/dl), the test compound (650 pmole) solubilized in solvent (95 % propylene glycol + 5 % ethanol) was subcutaneously injected at the behind of the rats in a dose of 0.1 ml per day. In a control group, only the solvent was injected lilkewise. Three and four days after the first injection, the blood was taken and the calcium concentration in the serum was measured, and the obtained data were shown in the average thereof.

Test compounds: The same as used in the above Experiment 1.

Results: The test results are shown in the following Table 2.

**Table 2**

| Test compouds | Increase of serum calcium concentration* (mg/dl) |
|---|---|
| 1α,25-dihydroxyvitamin D₃ | 3.1 ± 0.6 |
| Compound A | 0.7 ± 0.3 |
| Compound B | 0.6 ± 0.3 |

| | |
|---|---|
| *) It shows the data increased over the value (4.5 mg/dl) in the control group. | |

As is clear from the above test results, the compounds of the present invention showed less increase of the serum calcium concentration in comparison with the known 1α,25-dihydroxyvitamin D₃.

## Claims

1. A fluorine-containing vitamin D₃ analogue of the formula [I]: 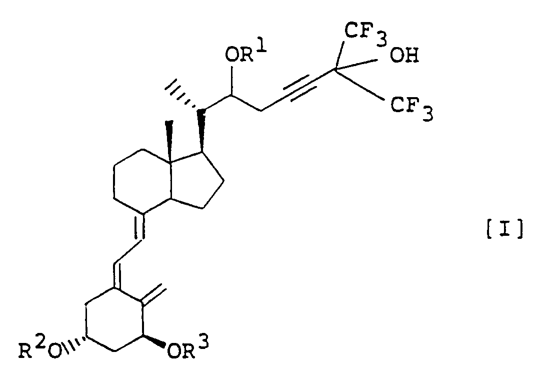 wherein R¹, R² and R³ are independently hydrogen atom or a hydroxy-protecting group.

2. The compound according to claim 1, wherein the hydroxy-protecting group is selected from the group consisting of methoxymethyl, ethoxyethyl, methoxyethoxymethyl, tetrahydropyranyl, trimethylsilyl, t-butyldimethylsilyl, t-butyldiphenylsilyl, acetyl

3. 26,26,26,27,27,27-Hexafluoro-24-homo-24-yne-1α,22S,25-trihydroxyvitamin D₃, or 1α,3-bis(t-butyldimethylsilyl) ether 22S-acetate thereof.

4. 26,26,26,27,27,27-Hexafluoro-24-homo-24-yne-1α,22R,25-trihydroxyvitamin D₃, or 1α,3-bis(t-butyldimethylsilyl) ether 22R-acetate thereof.

## Patentansprüche

1. Fluor enthaltende Vitamin-D₃-Analoge der Formel [I]: 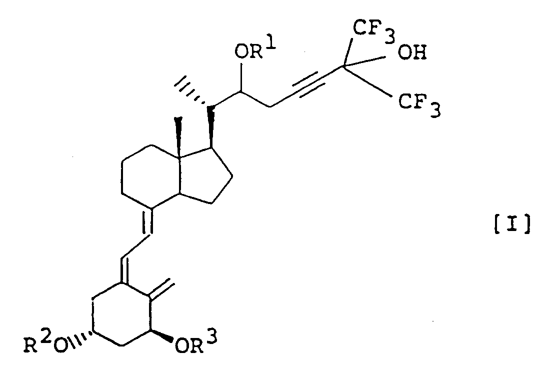 worin R¹, R² und R³ unabhängig ein Wasserstoffatom oder eine Hydroxy-Schutzgruppe bedeuten.

2. Verfahren nach Anspruch 1, worin die Hydroxy-Schutzgruppe ausgewählt wird aus der Gruppe, bestehend aus Methoxymethyl, Ethoxyethyl, Methoxyethoxymethyl, Tetrahydropyranyl, Trimethylsilyl, t-Butyldimethylsilyl, t-Butyldiphenylsilyl, Acetyl.

3. 26,26,26,27,27,27-Hexafluor-24-homo-24-in-1α,22S,25-trihydroxyvitamin D₃ oder das 1α,3-Bis(t-butyldimethylsilyl)ether-22S-acetat davon.

4. 26,26,26,27,27,27-Hexafluor-24-homo-24-in-1α,22R,25-trihydroxyvitamin D₃ oder das 1α,3-Bis(t-butyldimethylsilyl)ether-22R-acetat davon.

## Revendications

1. Analogue de la vitamine D₃ contenant du fluor de formule [I] : 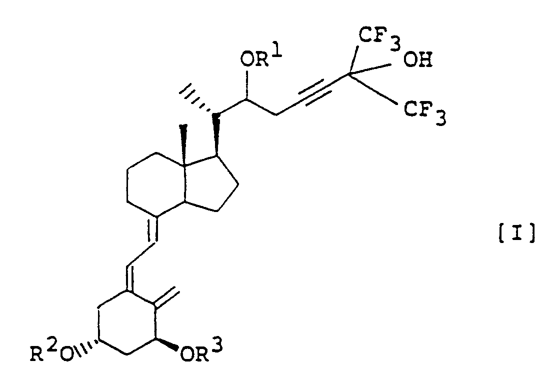 dans laquelle R¹, R² et R³ représentent indépendamment un atome d'hydrogène ou un groupement protecteur de l'hydroxyle.

2. Composé selon la revendication 1, dans lequel on choisit le groupement protecteur de l'hydroxyle dans le groupe constitué du méthoxyméthyle, de l'éthoxyéthyle, du méthoxyéthoxyméthyle, du tétrahydropyranyle, du triméthylsilyle, du t-butyldiméthylsylile, du t-butyldiphénylsilyle, de l'acétyle.

3. 26,26,26,27,27,27-hexafluoro-24-homo-24-yne-1α,22S,25-trihydroxyvitamine D₃ ou son 1α,3-bis(éther de t-butyldiméthylsilyle)-22S-acétate.

4. 26,26,26,27,27,27-hexafluoro-24-homo-24-yne-1α,22R,25-trihydroxyvitamine D₃ ou son 1α,3-bis(éther de t-butyldiméthylsilyle)-22R-acétate.
